# EUROPEAN PATENT SPECIFICATION

(11) **EP 2 755 991 B1**
(45) Date of publication and mention of the grant of the patent: **16.05.2018**
(21) Application number: 12832649.3
(22) Date of filing: 07.09.2012
(51) Int. Cl.: C07J 63/00

(54) **METHOD FOR PREPARATION OF BETULINIC ACID**
VERFAHREN ZUR HERSTELLUNG VON BETULINSÄURE
PROCÉDÉ DE PRÉPARATION D'ACIDE BÉTULINIQUE

(30) Priority: 12.09.2011 SE 1150819
(43) Date of publication of application: 23.07.2014
(73) Proprietor: Stora Enso Oyj, 00101 Helsinki (FI)
(72) Inventor: TULISALO, Jukka, FIN-04400 Järvenpää (FI); PIRTTIMAA, Minni, FIN-02600 Espoo (FI); ALAKURTTI, Sami, FIN-01450 Vantaa (FI); YLI-KAUHALUOMA, Jari, FIN-00790 Helsinki (FI); KOSKIMIES, Salme, FIN-00850 Helsinki (FI)
(74) Representative: Steinrud, Henrik
(86) International application number: PCT/IB2012/054638
(87) International publication number: WO 2013/038314

(56) References cited:
- WO-A1-2006/063464
- WO-A1-2006/105354
- WO-A2-2006/105357
- WO-A2-2006/133314
- RU-A- 2003 119 067
- US-A1- 2003 073 858
- US-A1- 2003 073 858
- HOLY, J. ET AL.: 'Dimethylaminopyridine derivatives of lupane triterpenoids are potent disruptors of mitocondrial structure and function' BIOORG. MED. CHEM. vol. 18, 2010, pages 6080 - 6088, XP027192851
- BARTHEL, A. ET AL.: 'Oxidative transformations of betulinol' TETRAHEDRON vol. 64, 2008, pages 9225 - 9229, XP023904166

## Description

### Field of the invention

The invention relates to catalytic, environmentally benign oxidation of betulin leading to synthesis of betulonic and betulinic acid.

### State of the art

Betulin has the structure 1, shown below, and is a naturally occurring pentacyclic terpene alcohol of lupane family, also known as betulinol and lup-20(29)-ene-3β,28-diol. Betulin is found in the bark of some tree species, particularly in the birch (*Betula* sp.) bark at best amounts up to 40% of the birch outer bark dry weight. In addition to betulin, also minor amounts of betulin derivatives are obtained from tree bark. There are known methods mainly based on extraction for isolation of betulin from bark material.

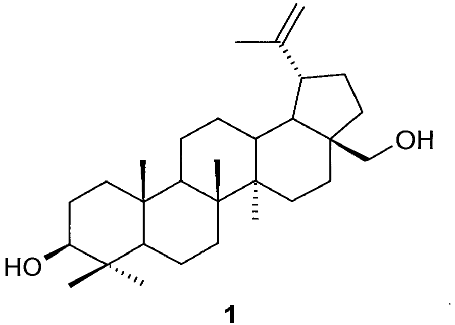

In some applications, poor solubility of betulin causes problems with respect to use and formulation, and accordingly, betulin is converted to its derivatives to improve solubility as well as bioactivity. In recent years it has been found, that especially betulinic acid and betulonic acid seem to possess high bioativities in several applications. This has caused the need to develop feasible and environmental benign catalytic methods for oxidation of betulin to betulinic acid and betulonic acid. In the production of said derivatives, reactivities of the functional groups of betulin, that is, primary and secondary hydroxy groups and the double bond are typically utilized.

Suitability of betulin and the derivatives thereof for medical and cosmetic applications and for industrial chemical applications is known to some extent. Use of betulinic acid in cosmetic applications such as promoters of growth and as components in skin creams is known from WO 0003749. The publication of WO 0174327 discloses the use of betulinic acid in sun creams for prevention of detrimental effects of the UV-light. The publication of WO 07141389 discloses the use of betulonic acid in several cosmetic applications and in WO 07141391 is described the use of betulonic acid and some betulin derivatives as antiprotozoal agent. The publication of WO 07141383 discloses the use of betulinic and betulonic acid as antifeedant agent for best management.

Betulinic acid having the structure of 3 shown in the reaction scheme A below may be isolated e.g. from birch (*Betula* sp.) bark or cork of cork oak (*Quercus suber* L.) by extraction, and further may be produced by several methods mainly based on direct oxidation of the betulin or birch bark material. The reaction scheme A shows the direct oxidation of the betulin **1** according to US 6,280,778 as Jones oxidation in the presence of chromium(VI) oxide catalyst to give betulonic acid 2, followed by the selective reduction of betulonic acid **2** thus obtained with sodium hydroxide to give betulinic acid **3.**

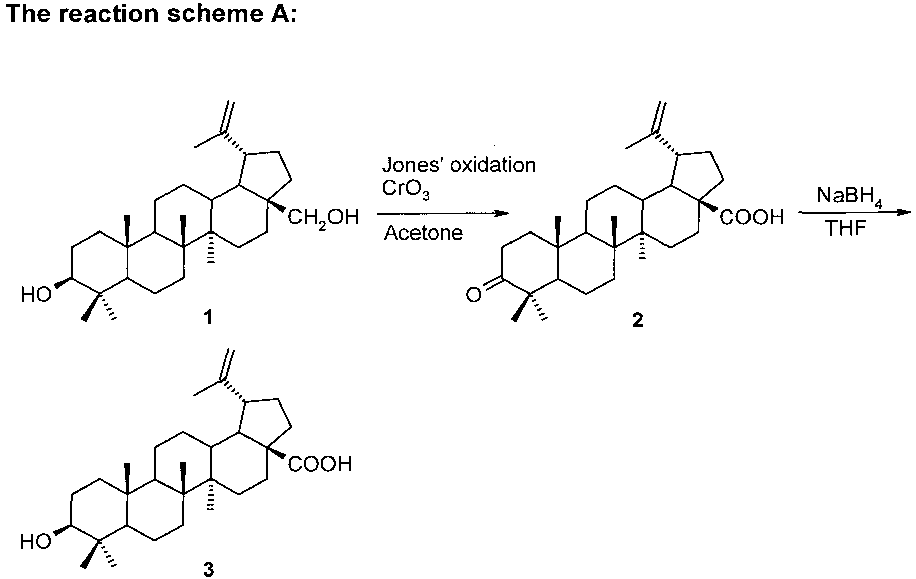

The problem with this method is that chromium(VI) oxide catalyst is carcinogenic and the reaction cumulates also large amounts of waste.

The alternative process for the production of betulinic acid is disclosed in US 5,804,575, comprising an oxidation step where 3-beta-hydroxyl of betulin is protected by acetylation. Isomerization and oxidation of secondary hydroxyl group of betulin is thus prevented. However, protecting the 3-beta-hydroxyl brings a new process stage for this method, since the protective group has to be removed for synthesizing free betulinic acid.

Most relevant prior art to present invention have been presented in US 20030073858 and WO 2006/13314.

US 20030073858 discloses a method for preparing first betulinic aldehyde and then betulinic acid from betulin. This method requires first protecting 3-OH-goup of betulin (by acylation) and only after this, it is possible to prepare corresponding 3 -protected betulinic aldehyde and then betulinic acid. Protective group in 3 -position should be cleaved before forming betulinic aldehyde and then betulinic acid.

WO 2006/13314 discloses a method for converting betulin to 3B-protected betulinic aldehyde and further to 3B-protected betulinic aldehyde. This reaction requires protecting 3B-keto group before preparing betulonic acid from-betulonic aldehyde. Protective group should be removed after betulonic acid has been prepared. This document also discloses preparation of betulonic aldehyde from betulin but not by direct catalytic conversion.

### The objects of the invention

The object of the present invention is to get rid of the drawback of the above mentioned prior art:
The first object is to develop environmentally benign method for oxidation betulin to betulinic and/or betulonic acid.

The second object is to develop an industrial-scale oxidation process for oxidation of betulin into betulinic and/or betulonic acid with a good yield and low costs.

The third object is to develop an oxidation process of betulin into betulinic and/or betulonic acid in which the product is ready-to-use without excessive purification also for medical and cosmetic applications.

The fourth object is to develop a synthesizing method for betulinic and/or betulonic acid which would require as few process stages as possible.

### Description of the invention

The above mentioned objects can be achieved by the method according to claim 1.

The claimed method relates to preparation of betulonic acid and/or betulinic acid from betulin wherein the method comprises a step of oxidizing betulin first to betulonic aldehyde or betulinic aldehyde with Pd(II)-catalyst catalyzed oxidation process, in the presence of dioxygen.

Dioxygen means here O₂ which is present at atmosphere or O₂, which has about the same activity as O₂ present in atmosphere.

The general idea behind the invention is to first oxidize betulin in the presence of catalytic amount of palladium catalyst and dioxygen to betulonic aldehyde and possibly also to betulinic aldehyde and then to further oxidize betulonic aldehyde into betulonic acid and further to reduce betulonic acid into betulinic acid. The oxidation of betulin to betulonic aldehyde is a homogeneous palladium catalysis process. The palladium catalyst is used in catalytic amount and the palladium-moiety in palladium catalyst is reduced into palladium(0) during the oxidation of betulin to betulonic aldehyde or betulinic aldehyde. Palladium catalyst is regenerated by re-oxidising of palladium(0)-moiety into palladium(II)-moiety. Additionally, to our surprise, we have found that betulin can be oxidized with Pd(II) catalyst and air to betulonic aldehyde or betulinic aldehyde and further to betulonic acid or betulinic acid simultaneously without purification, by using additional oxidant in reactions step 2 or 5 of scheme 1 below.

The preparation of betulonic acid and/or betulinic acid from betulin according to above mentioned method, wherein the first step is done by catalytic oxidation by using palladium(II) catalyst, has a high conversion rate and high selectivity.

Due to a highly selective conversion and the possibility to regenerate palladium catalyst the reaction is environmentally benign and prepared betulinic acid or betulonic acid is pure enough to be used for instance in pharmaceutical or cosmetic applications.

According to one embodiment of the invention the betulin is first oxidized with a Pd(II) catalyst and air to betulonic aldehyde or betulinic aldehyde and the obtained betulonic aldehyde or betulinic aldehyde is oxidized without purification further to betulonic acid or betulinic acid by using additional oxidation agent. In practice the catalytic oxidation of betulin and oxidation of betulonic aldehyde or betulinic aldehyde to corresponding acids happen simultaneously, in one pot (at the same vessel). Betulonic acid can be reduced to betulinic acid using known reducing methods, for example, by using sodium borohydride (NaBH₄) -reduction.

Above mentioned so called "one one pot oxidation method" comprises following process stages:
(c1) oxidizing betulin to betulonic aldehyde in presence of a catalyst and an oxidant,
(c2) oxidizing said betulonic aldehyde to betulonic acid with an oxidizing system,
(c3) reducing said betulonic acid to betulinic acid by using reducing agent, or
(d1) oxidizing betulin to betulinic aldehyde in presence of a catalyst and an oxidant,
(d2) oxidizing said betulinic aldehyde to betulinic acid with an oxidizing system, wherein
   - in stage (c1) and (d1) catalytic oxidation of betulin is performed by a Pd(II)-catalyst in presence of dioxygen and
   - catalytic oxidation reaction of betulin to betulonic aldehyde or betulin to betulinic aldehyde in stages (c1) or (d1) and oxidizing of betulonic aldehyde to betulonic acid (stage c2) or betulinic aldehyde to betulinic acid (stage d2) are performed simultaneously, in the same reaction vessel.

Because in this "one pot oxidation method" obtained betulonic aldehyde or betulinic aldehyde is not isolated but used as such, this method has an advantage of being effective and economic method for preparation betulonic or betulinic acid from betulin. This embodiment of the inventive method may be suitable for a large scale conversion of betulin to betulinic acid or betulonic acid.

Neither above mentioned general method for synthesizing betulinic acid or betulonic acid nor above mentioned so called "one pot method", needs functional groups of betulin to be protected with protecting groups. Therefore neither cleavage of the protection groups is needed. Thus, the number of reaction steps is reduced.

Homogeneous palladium catalysis process means herein, that the only catalyst is palladium(II) catalyst without presence of any co-catalyst.

Preparation of betulinic acid and betulonic acid from betulin proceeds mainly according to following reaction scheme 1.

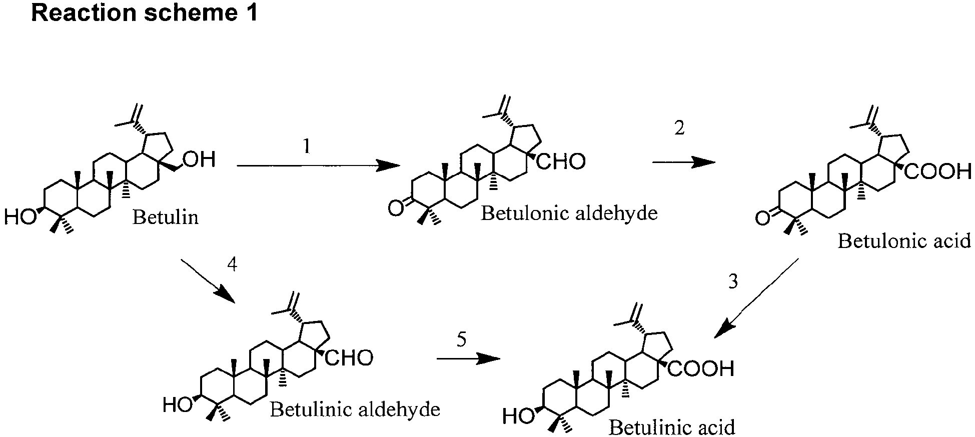

The catalytic oxidation of betulin to betulonic aldehyde proceeds via route 1 (to synthesize mainly betulonic aldehyde) or via route 4 (to synthesize mainly betulinic aldehyde). The catalytic oxidation of betulin to betulonic aldehyde or betulinic aldehyde is the first stage when preparing betulinic acid and/or betu-Ionic acid from betulin.

During the catalytic oxidation of betulin to betulonic aldehyde via route 1 it can also be obtained some amount of betulinic aldehyde, which can be oxidized directly to betulinic acid by reaction 5 (via route 5) of reaction scheme 1. Oxidation of betulonic aldehyde into betulonic acid proceeds via route 2 by using appropriate oxidizing system. Betulonic acid is then reduced to betulinic acid by the reaction 3 (via route 3).

The catalytic oxidation of betulin can also proceed via route 4, whereby betulinic aldehyde is obtained. Betulinic aldehyde can be directly oxidized via route 5 to betulinic acid using the same or different oxidation system than in above mentioned oxidation system used in oxidation of betulonic aldehyde into betu-Ionic acid by reaction 2.

The catalytic oxidation reaction from betulin to betulonic aldehyde by reaction 1 or 4 uses molecular dioxygen as the sole oxidant. Primary source of oxygen is air.

In the catalytic oxidation process of betulin using reaction 1 or 4, there can be used a variety of alternative palladium(II) catalyst. Palladium(II) catalyst is of the general formula (I):

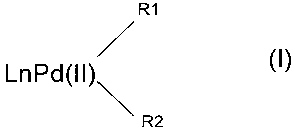

In the palladium catalyst of formula I:
n is any integer from 0 to 2, in a case n is 0 ligand L is absent;
L is any suitable ligand containing at least one nitrogen atom (N-ligand), which can be used for chelating palladium metal in palladium(II) catalyst. Suitable ligands can be found also from references Karimi, B and Zamani, A., Recent advances in the homogeneous palladium-catalyzed aerobioc oxidation of alcohols, J.Iran.Chem. Soc., Vol 5, Suppl., October 2008, pp. S1 - S20 or Gligorich, M and Sigman, M., recent advances and challenges of palladium(II)-catalyzed oxidation reactions with molecular oxygen as the sole oxidant, Chem.Commun., 2009, 3854-3867. Especially ligand may be selected from the group consisting of aromatic and aliphatic mono- and bidentate amines and mono- and bidentate heteroarenes. These may chelate palladium and form complexes with palladium(II) compounds. Ligand L includes bidentate pyridine and bidentate triethylamine and monodentate hydrotalcite.

The term "amine" refers herein to an amine group, which is originating to an ammonia group in which one, two or three hydrogen(s) may be substituted with a substituent. The substituent of an amine group is an aryl group in case of aromatic amines and an aliphatic alkyl group in case of aliphatic amines.

Aliphatic alkyl group means herein a substituent having a branched or unbranched saturated hydrocarbon chain from 1 to 10 carbon atoms, preferably 1 to 4 carbon atoms.

Aryl group may be an unsaturated aromatic carbocyclic group or also a heteroaryl group.

The unsaturated aromatic carbocyclic group has from 3 to 12 carbon atoms and has a single ring or multiple condensed rings, wherein at least one ring is aromatic, whereby said unsaturated aromatic carbocyclic group can be unsubstituted or substituted. Preferably unsaturated aromatic carbocyclic group is phenyl.

Heteroarene means herein a compound having monocyclic, bicyclic, or tricyclic ring system and containing one, two, or three aromatic rings and containing at least one nitrogen atom in aromatic ring, and which can be unsubstituted or substituted. Preferably heteroarene is pyridine.

Heteroaryl is a functional group, which is originating to a heteroarene.

Each symbol R1 or R2 mean a functional group selected independently from the group consisting of amines, aryls, carboxylates, esters, ketones, halogens and nitrates, wherein
The aryl group have the same definition as above, preferably aryl group means phenyl;
Carboxylate means an anion originating from carboxylic acid and which forms salt with palladium(II) cation, more preferable carboxylate is originating from a lower carboxylic acid such as acetic acid;
Ester means an alkylester, more preferable loweralkylester;
Ketone means an alkylketone, more preferable loweralkylketone or loweralkyldiketone, preferred loweralkyldiketone is actetylacetonate;
Preferred palladium(II) catalyst of general formula (I) include following catalysts in a case ligand is not present (n=0): palladium(II) acetate Pd(OAc)₂, phenylpalladium(II) acetate Pd(C₆H₅)(O₂CCH₃), palladium(II) acetylacetonate Pd(acac)₂, palladium(II) nitrate Pd(NO₃)₂.

Preferred palladium (II) catalysts of general formula (I), in which ligands are present (n=1 or n= 2) are those, wherein palladium(II) catalyst is of general formula (IA).

LₙPd(II)R₂ (IA)

Whereby the ligand L has the same definition as above and R have the same definition as R1 or R2 above.

The palladium(II) catalyst with formula (IA) comprises following preferable Pd(II) complexes: palladium(II)-hydrotalcite-complexes, palladium(II) compound - pyridine -complexes and palladium(II) compound-triethylamine (TEA)-complexes. These include following important palladium(II) catalysts:
(py)₂Pd(OAc)₂, (py)₂Pd(acac)₂, (py)₂Pd(NO₃)₂, (NEt₃)₂Pd(OAc)₂, (NEt₃)₃Pd(OAc)₂, wherein py means pyridine, Et means ethyl.

The first stage in reaction scheme 1, catalytic oxidation of betulin by palladium(II) catalyst and air to betulonic aldehyde or betulinic aldehyde, proceeds according to reaction step 1 or 4. It is performed in any suitable inert organic solvent in which at least betulin dissolves.

Inert solvent means herein that the solvent will not react essentially with betulin or its derivatives or reactants used in the methods according to reaction scheme 1 (for example oxidants).

Suitable inert solvents can be selected from the group comprising saturated or unsaturated aliphatic hydrocarbons, branched or unbranched aliphatic hydrocarbons, halogenated aliphatic hydrocarbons, aromatic hydrocarbons, fluorous solvents, chlorinated solvents, ethers, esters, ketones, H₂O, or mixtures thereof.

Preferable inert solvent is selected from the group consisting of toluene, xylene, decalin, DMSO, fluorous solvent, perfluoroalkane e.g. perfluorodecaline (can also improve O₂ solubility), THF, CH₂Cl₂, H₂O or mixtures thereof.

Most preferable inert solvents for this system are selected from the group consisting of aliphatic and aromatic hydrocarbons (eg toluene, xylenes, decalin), fluorous- and perfluoroalkanes (e.g. perfluorodecaline), chlorinated solvents (e.g. CH₂Cl₂) and ethers (eg. tetrahydrofuran).

The second stage for preparation of betulonic acid and/or betulinic acid from betulin proceeds via route 2 or 5 in reaction scheme 1. Betulonic aldehyde from reaction step 1 (via route 1) is oxidized into betulonic acid by reaction step 2 (via route 2) using suitable oxidizing system. Betulinic aldehyde from reaction 4 (via route 4) is oxidized into betulinic acid via route 5 using suitable oxidizing system.

If this second reaction stage is done immediately after or simultaneously to first reaction stage (after reaction step 1 or 4), the solvent used therein is preferably such that betulin and betulonic aldehyde and/or betulinic aldehyde will dissolve therein. In this case preferable solvent(s) are the same as above. If the second reaction stage (reaction step 2 or 5) is performed independently from the first reaction stage (reaction step 1 or 4), for instance in a separate vessel, the solvent may be any suitable organic solvent in which betulonic aldehyde or betulinic aldehyde will dissolve and which do not react with reactants.

The third stage of preparation of betulonic acid from betulin proceeds via route 3 (with reaction step 3) in reaction scheme 1. In this reaction step 3, betulonic acid is reduced into betulinic acid by using any suitable reduction agent such as NaBH₄, LiBH₄ or KBH₄. The reduction of betulonic acid to betulinic acid according to reaction step 3 can be performed also by using common methods known from the prior art.

There are many possible alternatives to be used as oxidizing systems in reaction steps 2 or 5. Such an oxidizing system includes an appropriate oxidizing agent and a possible buffer and possible auxiliary agents.

The used oxidizing agents can preferable be selected from the group consisting of: O₂, sodium hypochlorite (NaOCl), sodium chlorite (NaClO₂), potassium chlorite (KClO₂), potassium hypochlorite (KOCl), peroxides such as hydrogen peroxide (H₂O₂), hypervalent iodine reagents (eg. 2-iodoxybenzoic acid (IBX), 2-iodoxybenzenesulfonic (IBS)), Oxone (2KHSO₅, KHSO₄, K₂SO₄) and phthalimides such as *N*-hydroxyphthalimide and mixtures thereof. As to other possible oxidizing agents, we refer here to literature of this field. In a case betulinic aldehyde is synthesized by a catalytic oxidation of betulin (via route 4 in reaction scheme 1), this can be conversed directly into betulinic acid by oxidizing betulinic aldehyde.

In combination with above mentioned oxidizing agents it is often useful to use suitable buffer in an oxidation system. These buffers may be selected from the group consisting of:
KH₂PO₄, Na₂HPO₄, NaH₂PO₄, CHES, MES (*N*-morpholino)ethanesulfonic acid, TAPS([tris(hydroxymethyl)methyl]amino}propanesulfonic acid), Bicine (bis(2-hydroxyethyl)glycine), Tris (tris(hydroxymethyl)methylamine), Tricine (*N*-tris(hydroxymethyl)methylglycine), TAPSO (3-[*N-*Tris(hydroxymethyl)methylamino]-2-hydroxypropanesulfonic Acid), HEPES (4-2-hydroxyethyl-1-piperazineethanesulfonic acid), TES (2-{[tris(hydroxymethyl)methyl]amino}ethanesulfonic acid), MOPS (3-(*N-*morpholino)propanesulfonic acid) PIPES (piperazine-*N*,*N*'-bis(2-ethanesulfonic acid), Cacodylate (dimethylarsinic acid), SSC (saline sodium citrate) and MES (2-(*N*-morpholino)ethanesulfonic acid).

In the following are given pH-ranges for several above mentioned buffers KH₂PO₄, pH 5.8 - 8, NaH₂PO₄, pH 6 - 7.5 and CHES, pH 8.6-10.

In the following are given p*K*ₐ at 20°C Δp*K*ₐ/°C for several above mentioned buffers : MES 6.15 -0.011, ADA 6.6 -0.011, PIPES 6.8 -0.0085, ACES 6.9 - 0.020, Cholamine chloride 7.1 -0.027, BES 7.15 -0.016, TES 7.5 -0.020, HEPES 7.55 -0.014, Acetamidoglycine 7.7 -, Tricine 8.15 -0.021, Glycinamide 8.2 -0.029, Bicine 8.35 -0.018.

In the following are given p*K*ₐ at 25°C, (Buffer Range Temperature Effect), dpH/dT in (1/K) ** Mol Weight and Full Compound Name for several above mentioned buffers:
TAPS 8.43 7.7-9.1 -0.018 243.3 3-{[tris(hydroxymethyl)methyl]amino}propanesulfonic acid,
Bicine 8.35 7.6-9.0 -0.018 163.2 *N*,*N*-bis(2-hydroxyethyl)glycine,
Tris 8.06 7.5-9.0 -0.028 121.14 tris(hydroxymethyl)methylamine,
Tricine 8.05 7.4-8.8 -0.021 179.2 *N*-tris(hydroxymethyl)methylglycine,
TAPSO 7.635 7.0-8.2 259.3 3-[*N*-Tris(hydroxymethyl)methylamino]-2-hydroxypropanesulfonic Acid,
HEPES 7.55 6.8-8.2 -0.014 238.3 4-2-hydroxyethyl-1-piperazineethanesulfonic acid,
TES 7.40 6.8-8.2 -0.020 229.20 2-{[tris(hydroxymethyl)methyl]amino}ethanesulfonic acid,
MOPS 7.20 6.5-7.9 -0.015 209.3 3-(*N*-morpholino)propanesulfonic acid ,
PIPES 6.76 6.1-7.5 -0.008 302.4 piperazine-*N*,*N*'-bis(2-ethanesulfonic acid),
Cacodylate 6.27 5.0-7.4 138.0 dimethylarsinic acid,
SSC 7.0 6.5-7.5 189.1 saline sodium citrate and
MES 6.15 5.5-6.7 -0.011 195.2 2-(*N*-morpholino)ethanesulfonic acid.

Above mentioned auxiliary agents for oxidation systems, using routes 2 or 5 in reaction scheme 1 (oxidation of betulonic aldehyde or betulinic aldehyde to corresponding acid), include possible palladium scavenging. These scavenging agents are used especially when betulonic acid is prepared so that reaction steps of 1 and 2 are performed simultaneously or betulinic acid is prepared so, that reaction steps of 4 and 5, in reaction scheme 1, are performed simultaneously (this is so called one pot oxidation method). When betulinic acid or betulonic acid is synthesized using above described one pot method, betulonic aldehyde or betulinic aldehyde which have been obtained from catalytic oxidation of betulin according to reaction step of 1 or 4 are used as such, that means, without isolating them, before using in subsequent reaction steps 2 and 5.

Regenerating palladium(II) catalyst enhances significantly overall process efficiency of the process according to reaction scheme 1. Regenerating catalyst is especially important in large scale production. There are many known palladium scavengers, which can be used after catalytic oxidation process via route 1 or 4 for scavenging used palladium(0) compounds. Palladium scavengers which can be used for scavenging used palladium(0) compounds are known from the literature of this field. These suitable palladium(0) scavengers include free, polymer bound, silica supported or functionalized silica scavengers. Suitable functional groups in palladium(0) scavengers includes thiol, thiourea, cysteine, dimercaptotriazine (DMT), amine, diamine, triamine, imidazole, triaminetetraacetic Acid (TAAcOH), triaminetetraacetate sodium salt (TAAcONa), for example, but not limited to, 3-(diethylenetriamino)propyl, DL-dithiothreitol, ethylenediaminetriacetic acid acetamide, N,N,N'-trimethylethylenediamine, 6-thionicotinamide, bis-[(diphenylphosphanyl)-methyl]amine, 2-mercaptoethylamine, bipyridine, 3-(ethylenediamino)propyl, 3-mercaptopropyl, 2-(4-(ethylenediamino)benzyl)ethyl, 3-(1-thioureido)propyl, *N*-acetyl-L-cysteine, mercaptophenylaminobut-2-enoate ester, trimercaptotriazine, triamine tetraacetate and triamine tetracetate sodium salt.

In one embodiment of the invention palladium scavenger is *N*-acetyl-L-cysteine (used 11-fold excess) or QuadraPure MPA (mercaptophenylamino-but-2-enoate ester).

Above mentioned auxiliary agents for oxidation can also include oxidation preventative agents which will prevent undesirable oxidation of betulin or its derivatives (betulonic aldehyde or betulinic aldehyde).

Oxidation preventative agents are well known in this field and they are usually compounds, which will itself become reversible or irreversible oxidized. A typical oxidation preventative agents include olefins such as 2-methylbutene or more electron-rich reaction partner as sacrificial electrophile scavenger (e.g. resorcinol, 1,3-dihydroxybenzene).

Pd catalyst may be bound on a conventional catalyst support such as zeolite, alumina, carbon, or the like.

Molecular sieves, preferably 3 Å MS, can be optionally used to enhance the rate of the Pd(OAc)2-pyridine catalysed oxidation and to improve catalyst stability by decrasing precipitation of Pd(0).

In connection with the present invention, various additives and measurements can be useful to improve process efficiency. These additives include phase transfer catalysts, if a heterogeneous reaction mixture is used in any of the routes 1, 2, 3, 4 or 5 in reaction scheme 1. Phase transfer catalysts are used in heterogeneous reaction mixtures to facilitate movement of a reactant from one phase to another. Phase transfer catalysts are also known to sometimes accelerate reaction rates and minimize solvent waste, since the reactions tend to be heterogeneous. Herein used phase transfer catalyst may be selected from the group consisting of: ammonium salts, heterocyclic ammonium salts and phosphonium salts. Preferable phase transfer catalysts can be selected from the following group comprising of: tert-butanol, 1,2,3-trimethylimidazolium methyl sulfate, 1-butyl-2,3-dimethylimidazolium chloride, 1-butyl-2,3-dimethylimidazolium hexafluorophosphate, 1-butyl-2,3-dimethylimidazolium tetrafluoroborate, 1-ethyl-2,3-dimethylimidazolium ethyl sulphate, 1-methylimidazolium hydrogen sulfate, methyltrioctylammonium thiosalicylate, tetrabutylammonium heptadecafluorooctanesulfonate, tetrabutylammonium nonafluorobutanesulfonate, tetraphenylphosphonium chloride, tributylmethylammonium methyl sulphate, trihexyltetradecylphosphonium bis(2,4,4-trimethylpentyl)phosphinate, trihexyltetradecylphosphonium bromide, trihexyltetradecylphosphonium chloride, trihexyltetradecylphosphonium dicyanamide, hexadecyltrimethylammonium bromide, tetrabutylammonium hydroxide, tetraethylammonium chloride hydrate, tributylhexadecylphosphonium bromide, (-)-*N*,*N*-dimethylephedrinium bromide, (-)-*N*,*N*-dimethylephedrinium bromide, (-)-*N*-dodecyl-*N*-methylephedrinium bromide, benzyltributylammonium chloride, hexyltributylphosphonium bromide, methyltrialkyl(C8-C10)ammonium chloride, hexyltributylphosphonium bromide, *N-*benzylcinchoninium chloride, tetrahexylammonium hydrogensulfate, tetraoctylammonium hydroxide, (1*R*,2*S*)-(-)-*N*-methylephedrine, 3-bromo-2-methylpropene, *N*-(diphenylmethylene)aminoacetonitrile, bis(triphenylphosphoranylidene)ammonium chloride, tricaprylylmethylammonium chloride, riethylbenzylammonium chloride (TEBAC), tetrabutylammonium bromide (TBAB), tetraethylammonium bromide (TEAB), tetrabutylammonium hydrogensulfate, ethyltriphenylphosphonium iodide, methyltriphenylphosphonium iodide, ethyltriphenylphosphonium bromide, methyltriphenylphosphonium bromide, tetramethylammonium hydroxide, methyl trioctyl ammonium hydrogen sulfate (MTOAHS) and dimethyl dioctadecyl ammonium chloride (Arquad 2HT).

Especially following oxidizing systems (oxidizing agent + buffer + possible auxiliary agents + possible additive) has proven to be useful herein:
NaClO₂ + NaH₂PO₄ + 2-methylbutene + *tert*-butanol,
NaClO₂ + NaH₂PO₄ + 2-methylbutene + dimethyldioctadecylammonium chloride (arquad 2HT)
NaClO₂ + NaH₂PO₄ + 2-methylbutene + methyl trioctyl ammonium hydrogen sulfate (MTOAHS)
NHPI (N-hydroxyphthalimide) + air and
QuadraPure MPA (mercaptophenylaminobut-2-enoate ester) + air.

These all are suitable for a method, in which routes 1 and 2 or 4 and 5 are performed simultaneously using so called "one pot oxidation method".

Measurements which will facilitate oxygen dissolving into reaction mixture may also be of the advantage in oxidation reactions via route 1, 2, 4 or 5. Such measurements include for example vigorous stirring of reaction vessel and performing oxidation process under overpressure (up to about 100 atm).

### Examples

General procedure for oxidation of betulin with Pd(II) catalyst includes loading of reactor with inert solvent and connecting air flow and stirring to the system and adding then Pd-catalyst (2-10 mol-% from the amount of betulin) and N-ligand (10-2000 mol-%) and heating the reactor to 50-120 °C. Then betulin is added in small portions within 1-2 hrs and reaction is allowed to proceed for 5-15 hrs at 50-120 °C. Then Pd recycling agent, eg. *N*-acetyl-L-cysteine (11-fold excess) or QuadraPure MPA (mercaptophenylaminobut-2-enoate ester) is added and adsorbed Pd is allowed to precipitate while cooling the reaction to 5-25 °C and precipitate is removed by filtration. The filtrate contains betulonic aldehyde, crude yield 83-92% based on betulin.

According to this invention betulonic aldehyde can be further oxidized to betu-Ionic acid by adding additional oxidant to filtrate containing betulonic aldehyde and stirring the reaction typically at 5-50 °C for 1- 25 hrs, depending the type of additional oxidant system. The overall isolated yield of 48-76 % for betu-Ionic acid (purity >90%) based on betulin is obtained.

### Examples concerning to step 1 for catalytic oxidation of betulin to betu-Ionic aldehyde

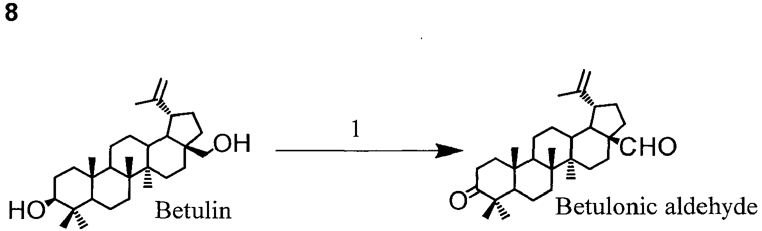

This reaction step was performed using palladium (II) catalyst containing Pd(OAc)₂ + pyridine or catalyst comprising Pd-hydrotalcite and air as an oxidizing agent. Pd(OAc)₂ = Palladium(II) acetate;

### Example 1.1

### Pd(OAc)₂+pyridine+air (N-acetyl-L-cysteine as Pd scavenger)

**Betulonic aldehyde.** Toluene (500 ml) was added to a 1 l flask equipped with condenser, mechanical stirring and tubing for air bubbling. Palladium(II) acetate (1.2 g, 6 w-%) and pyridine (5 ml) were added. Temperature was adjusted to 80 °C, air flow to the reaction mixture was adjusted to 1000 ml/min and stirring to 500 rpm. Betulin (20.0 g) was added in small proportions during 1 h. Oxidation reaction was monitored by GC and after 7 hour reaction was finished. *N*-acetyl-L-cysteine (8.0 g) was added to scavenge Pd(II). Reaction mixture was cooled down and stirred under N₂ flow for 1 h. Residue was filtered with vacuum and washed with toluene (3 × 20 ml). Obtained filtrate (including betulonic aldehyde 88%) was subsequently used in the next reaction step (2.1, 2.3 and 2.3) in one pot without further purification.

XRF-analysis of dried precipitate: 5.7% Pd (resulting 85% Pd recovery).

GC analysis of filtrate: Betulin < 0.1%, betulonic aldehyde 88%, betulinic aldehyde 2%, others 10%.
¹H NMR (300 MHz, CDCl₃): 0.92 (s, 3H), 0.94 (s, 3H), 0.97 (s, 3H), 1.01 (s, 3H), 1.06 (s, 3H), 1.69 (s, 3H), 2.43 (m, 2H), 2.86 (m, 1H), 4.62 (s, 1H), 4.75 (s, 1H), 9.66 (s, 1H)
¹³C NMR (75 MHz, CDCl₃): 14.1, 15.6, 15.9, 18.9, 19.5, 21.0, 21.2, 25.4, 26.5, 28.7, 29.1, 29.8, 33.1, 33.5, 34.1, 36.8, 38.7, 39.6, 40.7, 42.5, 47.3, 47.4, 47.9, 49.7, 54.9, 59.2, 110.2, 149.6, 206.5, 218.0

### Example 1.2

### Pd(OAc)₂+pyridine+air (No Pd scavenger)

**Betulonic aldehyde.** Toluene (30 ml) was added to a 100 ml flask equipped with a condenser, mechanical stirrer and tubing for air bubbling. Palladium(II) acetate (26 mg, 5 w-%) and pyridine (0.1 ml) were added. Temperature was adjusted to 80 °C, air flow to 50 ml/min and stirring to 500 rpm. Betulin (0.50 g) was added. Oxidation reaction was monitored by GC and after 7 hour reaction was finished. Obtained solution (including betulonic aldehyde 88%) was subsequently used in the next reaction step in one pot without further purification.

### Examples relating to step 2 for preparing betulonic acid

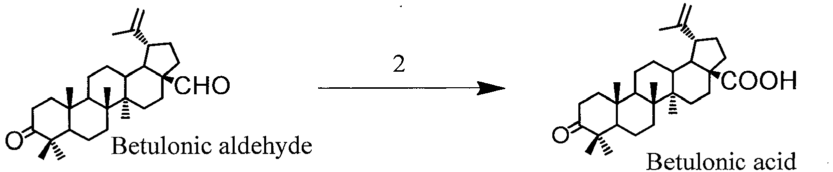

### Oxidation of betulonic aldehyde to betulonic acid using various oxidation systems

### Example 2.1

### 2-methylbutene+NaClO₂+NaH₂PO₄+ tert-butanol

**Betulonic acid.** T*ert*-butanol (170 ml) and 2-methyl-2-butene (30 ml) were added to the filtrate obtained from example 1 (including 20 g of betulonic aldehyde, purity 85-90%) under N₂ flow. The oxidation system comprising NaClO₂ (17.0 g) and NaH₂PO₄*H₂O (25.2 g) in water (200 ml) were added during 0.5 h and stirring was continued at room temperature under N₂ for 15 h. Organic and aqueous phases were separated and organic phase was washed with water (200 ml). Aqueous NaOH (2.7 g in 40 ml of water) was added to organic phase and stirred for 0.5 h. Half of the solvent was evaporated off under vacuum and residue was stirred at room temperature for 1 h. Precipitated betulonic acid Na-salt was filtered, washed with toluene (4 × 20 ml) and dried overnight at vacuum to yield betulonic acid Na-salt (16.6 g). Betulonic acid Na-salt was subsequently treated aqueous HCI solution (140 ml water and 60 ml of 10% HCI) and stirred for 2.5 h. Precipitate was filtered and washed with water (5 × 30 ml), dried in vacuum oven at 60 °C overnight to obtain betulonic acid 14.9 g (73% from betulin).
GC: betulonic acid 92%, betulinic acid 2%, others 6%.
¹H NMR (300 MHz, CDCl₃): 0.93 (s, 3H), 0.98 (s, 3H), 0.99 (s, 3H), 1.02 (s, 6H), 1.07 (s, 3H), 1.70 (s, 3H), 1.96 (m, 3H), 2.27 (m, 2H), 2.44 (m, 2H), 3.01 (m, 1H), 4.62 (s, 1H), 4.74 (s, 1H).
¹³C NMR (75 MHz, CDCl₃) 14.6, 15.8, 15.9, 19.3, 19.6, 21.0, 21.3, 25.5, 26.6, 29.6, 30.5, 32.1, 33.6, 34.1, 36.9, 37.0, 38.5, 39.6, 40.6, 42.5, 46.9, 47.3, 49.1, 49.8, 54.9, 56.3, 109.7, 150.3, 181.9, 218.4
NaClO₂ = Sodium chlorite
NaH₂PO₄ = Sodium dihydrogen phosphate

### Example 2.2.

### 2-methylbutene+NaClO₂+NaH₂PO₄+MTOAHS

**Betulonic acid.** Methyl trioctyl ammonium hydrogen sulphate (MTOAHS) (40 mg) and 2-methyl-2-butene (1 ml) were added to the filtrate obtained from example 1.1 (including 1.0 g of betulonic aldehyde, purity 85-90%) under N₂ flow. Oxidation system comprising NaClO₂ (0.5 g) and NaH₂PO₄*H₂O 0.2 M in water (20 ml) was added and stirring was continued at room temperature, under N₂ for 22 h. Organic and aqueous phases were separated and organic phase was washed with water (20 ml). Aqueous NaOH 1.7 M (4 ml) was added to organic phase and stirred for 0.5 h. Precipitated betulonic acid Na-salt was filtered, washed with toluene (10 ml) and dried in a vacuum oven at 80 °C. Betulonic acid Na-salt was subsequently treated with acetic acid (0.4 ml) in MeOH (8 ml) and stirred for 15 min. Water (15 ml) was added and precipitated betulonic acid was filtered and washed with water (4 × 10 ml), dried in the vacuum oven at 80 °C overnight. Isolated yield 0.34 g, GC purity analysis: betulonic acid 88%.

### Example 2.3.

### 2-methylbutene+NaClO₂+NaH₂PO₄+TBAHS

**Betulonic acid.** TBAHS (0.04 g) (tetrabutylammonium hydrogensulphate) and 2-methyl-2-butene (3 ml) were added to the filtrate obtained from example 1.1 (including 1.0 g of betulonic aldehyde, purity 85-90%) under N₂ flow. NaClO₂ (0.8 g) and NaH₂PO₄*H₂O 0.3 M in water (20 ml) were added and stirring was continued at room temperature, under N₂ for 16 h. GC purity analysis: betulonic aldehyde 32%, betulonic acid 20%, others 48%.

### Example 2.4.

### 2-methylbutene+NaClO₂+NaH₂PO₄+Arquad 2HT

**Betulonic acid.** Arquad 2HT (0.12 g) (Dialkyldimethylammonium acetate) and 2-methyl-2-butene (3 ml) were added to the filtrate obtained from example 1.1 (including 1.0 g of betulonic aldehyde, purity 85-90%) under N₂ flow. Oxidation system NaClO₂ (0.8 g) and NaH₂PO₄*H₂O 0.3 M in water (25 ml) was added and stirring was continued at room temperature under N₂ for 24 h. Organic and aqueous phases were separated and organic phase was washed with water (15 ml). Aqueous NaOH 1.7 M (4 ml) was added to organic phase and stirred for 1.5 h. Precipitated betulonic acid Na-salt was filtered, washed with toluene (10 ml) and dried. Betulonic acid Na-salt was subsequently treated with acetic acid (0.4 ml) in MeOH (8 ml) and stirred for 15 min. Water (15 ml) was added and precipitate of betulonic acid was filtered and washed with water (4 × 10 ml), dried in vacuum oven at 80 °C overnight. Isolated yield 0.58 g, GC purity analysis: betulonic acid 83%.

### Example 2.5.

### NHPI+air

Filtrate obtained from example 1.1 (including 1.0 g betulonic aldehyde, purity 85-90%) was cooled down to 0-5 °C under air flow of 50 ml/min. Methanol (0.2 ml) and oxidation system comprising N-hydroxyphthalimide (8.5 mg, NHPI) were added. The mixture was stirred at 0-5 °C for 2.5 hours after which NaOH 1.7 M in water (0.9 ml) was added and the mixture was stirred at 0-5 °C for 1 hour. Formed precipitate betulonic acid Na-salt was filtered and washed with toluene (10 ml). Betulonic acid Na-salt was dissolved in methanol (5 ml) at 50 °C. Acetic acid (0.2 ml) was added and the mixture was stirred at 50 °C for 10 min. Water (20 ml) was added and the mixture was cooled to 5-10 °C. The precipitate was filtered, washed with water and dried to obtain betulonic acid (0.26 mg). GC purity analysis: 86% betulonic acid.

### Example 2.6

### MPA+air

Reaction mixture (including 0.5 g of betulonic aldehyde, purity 85-90%) obtained from example 1.1.1. was cooled to room temperature and Quadrapure MPA (0.18 g) (Pd-scavenger) was added and reaction mixture was bubbled with air (50 ml/min) and stirred for 18 h. Precipitate was filtered with vacuum and washed with toluene (3 × 20 ml). Aqueous NaOH 1.7 M (3 ml) was added and mixture was stirred for 45 min. Precipitated betulonic acid Na-salt was filtered, washed with toluene (10 ml) and dried. Betulonic acid Na-salt was subsequently treated with acetic acid (0.4 ml) in MeOH (8 ml) and stirred for 30 min. Water (15 ml) was added and precipitated betulonic acid was filtered and washed with water (4 × 10 ml), dried in vacuum oven at 80 °C overnight. Isolated yield 0.49 g, GC purity analysis: betulonic acid 87%.

### Example relating to step 3 for preparing betulinic acid

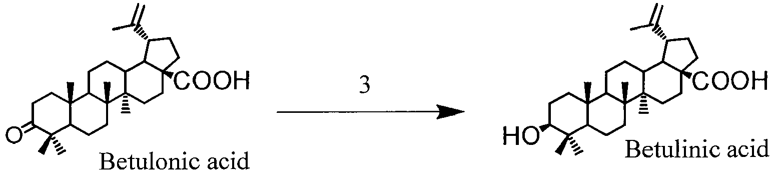

### Example 3.1.

### Reduction of betulonic acid to betulinic acid with NaBH₄

**Betulinic acid.** To a solution of water (130 ml), NaOH (26 ml, 5%) and 2-propanol (120 ml) was added betulonic acid (13.0 g, purity 94%). NaBH₄ (1.2 g) was added and reaction mixture was stirred for 3 hour in ice bath. Alkaline solution was acidified by adding aqueous HCI solution (130 ml, 10%). Precipitate betulinic acid was filtered, washed with water (4 × 50 ml) and dried in vacuum oven to yield betulinic acid 12.2 g (94%). GC: Betulinic acid 92%, betulonic acid < 0.1%, others 8%. To purify betulinic acid (92%) to very high purity (>99%), betulinic acid (92%) (5 g) was dissolved to refluxing ethanol and allowed to crystallize at 4 °C over night. Formed crystals were filtrated and dried in vacuum oven. Yield 82%, GC purity >99%.
¹H NMR (300 MHz, CDCl₃) 0.75 (s, 3H), 0.82 (s, 3H), 0.93 (s, 3H), 0.96 (s, 6H), 0.97 (s, 3H), 1.69 (s, 3H), 1.97 (m, 2H), 2.28 (m, 2H), 3.01 (m, 1H), 3.19 (dd, J = 5.5, 10.7 Hz, 1H), 4.60 (s, 1H), 4.74 (s, 1H);
¹³C NMR (75 MHz, DMSO) 14.4, 15.7, 15.8, 16.0, 18.0, 19.0, 20.5, 25.1, 27.2, 28.1, 29.2, 30.1, 31.7, 34.0, 36.4, 36.7, 37.6, 38.3, 38.5, 40.3, 42.0, 46.6, 48.6, 50.0, 54.9, 55.4, 76.8, 109.7, 150.3, 177.3
NaBH₄ = Sodium borohydride

### Example relating to step 4 for preparing betulinic aldehyde

### Example 4.1

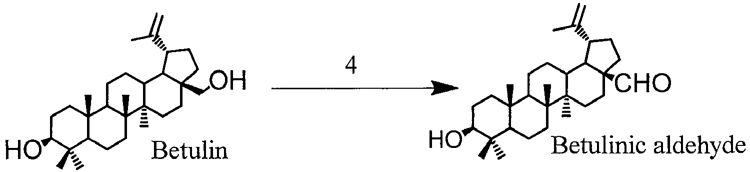

### Pd(OAc)₂+ triethyl amine (TEA) +air (N-acetyl-L-cysteine as Pd scavenger)

**Catalytic oxidation of betulin to betulinic aldehyde.** Mixture of betulin (2,00 g), Pd(OAc)₂ (120 mg), TEA triethylamine (500 mg) in THF (120 ml) was bubbled with air flow 500 ml/min with stirring at room temperature for 13 hour. Obtained solution mixture (including betulinic aldehyde 25%) was subsequently used in the next reaction step in one pot without further purification.
GC: Betulin 58%, betulinic aldehyde 25%, others 17%.
¹H NMR (500 MHz, CDCl₃): Aldehyde 45%.

### Example relating to steps 1 and 4 for manufacturing mixture of betulinic aldehyde and betulonic aldehyde

### Example 5.1

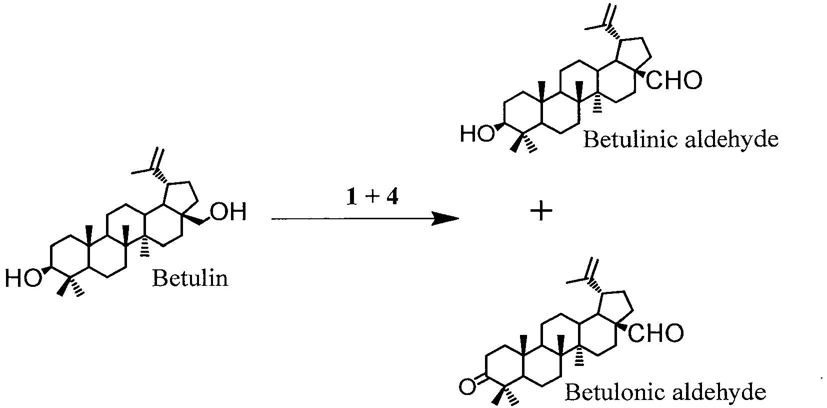

### Pd(OAc)₂+pyridine+air (MPI 167) (N-acetyl-L-cystine as Pd scavenger)

**Betulinic aldehyde and betulonic aldehyde.** Toluene (1000 ml) was added to a 2 l flask equipped with a condenser, mechanical stirrer and tubing for air bubbling. Palladium(II) acetate (2.43 g, 6 w-%) and pyridine (10 ml) were added. Temperature was adjusted to 80 °C, air flow to the reaction mixture was adjusted to 1000 ml/min and stirring to 500 rpm. Betulin (40.5 g) was added in small proportions during 1 h. Oxidation reaction was continued for 11 hour. N-acetyl-L-cystine (16.0 g) was added to scavenge Pd(II). Reaction mixture was cooled down and stirred under N₂ flow for 1 h. Residue was filtered with vacuum and washed with toluene (3 * 40 ml). Obtained filtrate (including betulonic aldehyde and betulinic aldehyde 80% together) was subsequently used in the next reaction step (5) in one pot without further purification.

### Example 5.2

**Formation of Pd(II)-hydrotalcite.** Mixture of Pd(OAc)₂ (378 mg), pyridine (333 mg) and hydrotalcite (10.0 g) in toluene (100 ml) was stirred at 80 °C for 1 hour. The mixture was cooled and formed Pd(II)-hydrotalcite precipitate (Pd-content 0.16 mmol/g) was separated by filtering, washed with diethyl ether (2×20 ml) and dried under vacuum at 25 °C.

### Pd(II) -hydrotalcite + pyridine + air

**Betulinic aldehyde and betulonic aldehyde.** Toluene (50 ml) was added to a 250 ml flask equipped with a condenser, mechanical stirrer and tubing for air bubbling. Pd(II)-hydrotalcite (0.7 g, 140 w-%) and pyridine (0.04 ml) were added. Temperature was adjusted to 80 °C, air flow to 50 ml/min and stirring to 700 rpm. Betulin (0.5 g) was added. Oxidation reaction was monitored by GC and after 7 hours reaction was finished. Reaction mixture was cooled down to room temperature, precipitate was filtered and washed with toluene (2×20 ml). Solvent was evaporated in vacuum and dried in a vacuum oven over night to yield mixture of betulin oxidation products.

GC: Betulin 1%, betulinic aldehyde 21%, betulonic aldehyde 28%, others 50%.

### Example relating to steps 2 and 5 for manufacturing mixture of betulinic acid and betulonic acid

### Example 6

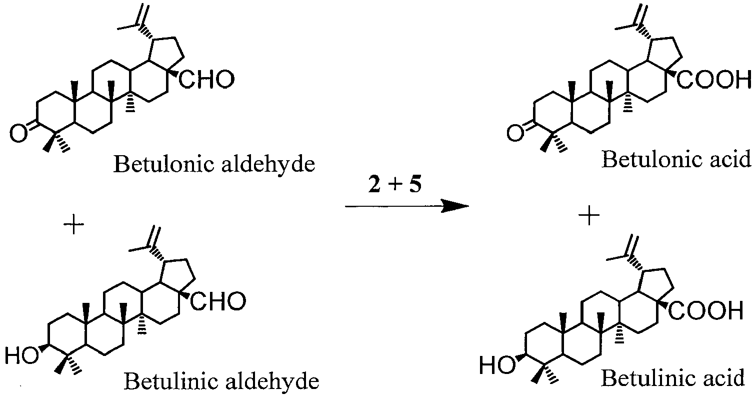

### 2-methylbutene+NaClO₂+NaH₂PO₄+ tert-butanol

**Betulinic acid and betulonic acid.** Tert-butanol (340 ml) and 2-methyl-2-butene (60 ml) were added to the filtrate obtained from example 4.2 (including 40 g mixture of betulinic aldehyde and betulonic aldehyde, aldehyde content 80%) under N₂ flow. The oxidation system comprising NaClO₂ (34.0 g) and NaH₂PO₄*H₂O (50 g) in water (400 ml) was added during 0.5 h and stirring was continued at room temperature under N₂ for 18 h. Organic and aqueous phases were separated and organic phase was washed with water (400 ml). Aqueous NaOH (5.4 g in 40 ml of water) was added to organic phase and stirred for 0.5 h. Half of the solvent were evaporated off under vacuum and residue was stirred at room temperature for 1 h. Precipitated mixture of betulinic acid and betulonic acid Na-salt was filtered, washed with toluene (4 * 40 ml) and dried over night at vacuum to yield mixture of betulinic acid and betu-Ionic acid Na-salt. Mixture of betulinic acid and betulonic acid Na-salt was subsequently treated with aqueous HCI solution (280 ml water and 120 ml of 10% HCI) and stirred for 2,5 h. Precipitate was filtered and washed with water (5 *60 ml), dried in vacuum oven at 60 °C over night to obtain mixture of betulinic acid and betulonic acid 17.8 g (44% from betulin).
GC: betulonic acid 60%, betulinic acid 27%, others 13%.

**Table 1. Results of betulin oxidation. Oxidation products of betulin. Route refers to reaction step 1,2, 3, 4 or 5 in above mentioned reaction scheme 1.**

| Route/ Catalyst | Oxidant | Betulinic aldehyde | Betulonic aldehyde | Betulonic acid | Betulinic acid |
|---|---|---|---|---|---|
| 1. Pd(OAc)₂ + pyridine | Air | 2 | 92 | - | - |
| 1. Pd(II)-hydrotalcite | Air | 21 | 28 | - | - |
| 2. NHPI | Air | | | 48 | |
| 2. QuadraPure MPA | Air | | | 62 | |
| 2. NaClO₂ + NaH₂PO₄ + 2-methyl butene + arquad 2HT | - | | | 68 | |
| 2. NaClO₂ + NaH₂PO₄ + 2- | - | | 4 | 76 | 2 |
| methylbutene + arquad 2HT + *tert-*butanol | | | | | |
| 3. NaBH₄ reduction | | | | | 94 |
| 4. Pd(OAc)₂ + TEA | | 25 | | | |
| 4. Pd(OAc)₂ + pyridine | | 25 | 55 | | |
| 5. NaClO₂ + NaH₂PO₄ + 2-methylbutene + arquad 2HT + *tert-*butanol | | | | 60 | 27 |

## Claims

1. A method for preparation of betulonic acid or betulinic acid from betulin wherein the method comprises process stages of:
(c1) oxidizing betulin to betulonic aldehyde in presence of a catalyst and an oxidant,
((c2) oxidizing said betulonic aldehyde to betulonic acid with an oxidizing system,
(c3) reducing said betulonic acid to betulinic acid by using reducing agent wherein
catalytic oxidation reaction of betulin to betulonic aldehyde in stage (c1) is performed simultaneously, in the same reaction vessel, with the oxidizing of betulonic aldehyde to betulonic acid in the stage (c2)
or
(d1) oxidizing betulin to betulinic aldehyde in presence of a catalyst and an oxidant,
((d2) oxidizing said betulinic aldehyde to betulinic acid with an oxidizing system, wherein
catalytic oxidation reaction of betulin to betulinic aldehyde in stage (d1) is performed simultaneously, in the same reaction vessel, with the oxidizing of betulinic aldehyde to betulinic acid in the stage (d2) and
- in stage (c1) or (d1) catalytic oxidation of betulin is performed by a Pd(II)-catalyst in presence of dioxygen.

2. The method according to claim 1, wherein dioxygen originates from atmospheric oxygen.

3. The method according to claim 1 or claim 2 , wherein Pd(II)-catalyst is of the general formula (I) 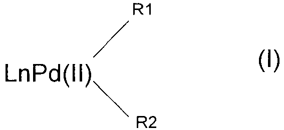 wherein
L is an aliphatic or heterocyclic ligand containing at least one nitrogen atom (N-ligand),
n is any integer from 0 to 2, in case n is 0 ligand L is absent;
R1 or R2 is a functional group selected independently from the group consisting of amines, unsaturated aromatic carbocyclic groups, carboxylates, esters, ketones, halogens and nitrate, wherein
unsaturated aromatic carbocyclic groups have from 6 to 12 carbon atoms and a single ring or multiple condensed (fused) rings, wherein at least one ring is aromatic and whereby said unsaturated aromatic carbocyclic group may be unsubstituted or substituted,
carboxylate means an anion originating from a carboxylic acid which forms a salt with palladium(II) cation, more preferably carboxylate originates from a lower carboxylic acid such as acetic acid, ester means an alkylester, and
ketone means an alkylketone, such as acetylacetonate.

4. The method according to claim 3, wherein ligand L is selected from the group consisting of aromatic and aliphatic mono- and bidentate amines (n=1 or 2) and mono- and bidentate (n=1 or 2) herearoarenes, whereby each of the aromatic amine groups originates from an ammonia group in which one, two or three hydrogen(s) is/are substituted with an aryl group, each of the aliphatic amine groups originates from an ammonia group in which one, two or three hydrogen(s) is/are substituted with an aliphatic alkyl group, whereby
aliphatic alkyl group means a substituent having branched or unbranched saturated hydrocarbon chain from 1 to 10 carbon atoms, preferably 1 to 4 carbon atoms and
aryl group means an unsaturated aromatic carbocyclic substituent having from 6 to 12 carbon atoms and having a single ring or multiple condensed rings, wherein at least one ring is aromatic, whereby said unsaturated aromatic carbocyclic group can be unsubstituted or substituted or
aryl group means a heteroaryl group which is derived from a monocyclic, bicyclic, or tricyclic heteroaerene ring system containing one, two, or three aromatic rings and containing at least one nitrogen atom in an aromatic ring, and which can be unsubstituted or substituted and
heteroarene group means a monocyclic, bicyclic, or tricyclic heteroaerene ring system containing one, two, or three aromatic rings and containing at least one nitrogen atom in an aromatic ring, and which can be unsubstituted or substituted.

5. The method according to any of the claims 3 or 4, wherein palladium catalyst (I) is selected from the group consisting of palladium (II) acetate Pd(OAc)₂, phenylpalladium(II) acetate Pd(C₆H₅)(O₂CCH₃), palladium(II) acetylacetonate Pd(acac)₂ and palladium(II) nitrate Pd(NO₃)₂.

6. The method according to claim 1, wherein palladium II) catalyst is of general formula (IA).
LₙPd(II)R₂ (IA)
Whereby the dedfinition of L has the same meaning as above and R have the same meaning as R1 or R2 above and n means 1 or 2.

7. The method according to any of the claims 3 - 6, wherein palladium(II)) catalyst of the formula (I) is selected from the group consisting of: palladium(II) compound immobilized on talcite, palladium (II)-hydrotalcite-complexes, palladium(II) compound - pyridine -complexes and palladium (II) compound-triethylamine (TEA)-complexes.

8. The method according to claim 7, wherein the palladium (II) catalyst is selected from the group consisting of: (py)₂Pd(OAC)₂, (py)₂ Pd(acac)₂, (py)₂ Pd(NO₃)₂, (Net₃)₂Pd(Oa_{C})₂, (Net₃)₃Pd(Oac)₂, wherein py means pyridine and et means ethyl.

9. The method according to any of the previous claims, wherein step of oxidizing betulin to betulonic aldehyde or betulinic aldehyde with Pd(II)-catalyzed oxidation in the presence of dioxygen is carried out in a solvent selected from the group consisting of aromatic hydrocarbons, ethers, halogenated aliphatic hydrocarbons, H₂O, or mixtures thereof.

10. The method according to claim 9 wherein solvent is CH₂Cl₂, DMF or THF, DMSO, H₂O, perfluoroalkane e.g. perfluorodecaline, toluene, xylene or mixtures thereof.

11. The method according to any of the previous claims wherein temperature during the oxidation of betulin is 50 -120 °C, preferably about 80 °C.

12. The method according to any of the previous claims, wherein palladium catalyst is regenerated after its Pd(II) -moiety has been reduced into Pd(0)-moiety during the oxidation of betulin to betulonic aldehyde and/or betulinic aldehyde.

13. The method according to claim 12 wherein palladium catalyst is regenerated by the means of N-acetyl-L-cysteine or mercaptophenylaminobut-2-enoate ester.

## Patentansprüche

1. Verfahren zur Herstellung von Betulonsäure oder Betulinsäure aus Betulin, wobei das Verfahren die Verfahrensstufen umfasst:
(c1) Oxidieren von Betulin zu Betulonaldehyd in Gegenwart eines Katalysators und eines Oxidationsmittels,
(c2) Oxidieren des Betulonaldehyds zu Betulonsäure mit einem oxidierenden System,
(c3) Reduzieren der Betulonsäure zu Betulinsäure durch Benutzen von Reduktionsmittel, wobei
die katalytische Oxidationsreaktion von Betulin zu Betulonaldehyd in Stufe (c1) in demselben Reaktionsgefäß gleichzeitig mit dem Oxidieren von Betulonaldehyd zu Betulonsäure in der Stufe (c2) durchgeführt wird,
oder
(d1) Oxidieren von Betulin zu Betulinaldehyd in Gegenwart eines Katalysators und eines Oxidationsmittels,
(d2) Oxidieren des Betulinaldehyds zu Betulinsäure mit einem oxidierenden System, wobei
die katalytische Oxidationsreaktion von Betulin zu Betulinaldehyd in Stufe (d1) in demselben Reaktionsgefäß gleichzeitig mit dem Oxidieren von Betulinaldehyd zu Betulinsäure in der Stufe (d2) durchgeführt wird und
- in Stufe (c1) oder (d1) die katalytische Oxidation von Betulin mittels eines Pd(II)-Katalysators in Gegenwart von Disauerstoff durchgeführt wird.

2. Verfahren nach Anspruch 1, wobei Disauerstoff aus atmosphärischem Sauerstoff stammt.

3. Verfahren nach Anspruch 1 oder 2, wobei der Pd(II)-Katalysator die allgemeine Formel (I) aufweist 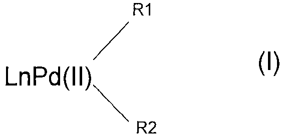 wobei
L ein aliphatischer oder heterocyclischer Ligand ist, der mindestens ein Stickstoffatom enthält (N-Ligand),
n eine beliebige ganze Zahl von 0 bis 2 ist, wobei in einem Fall, in dem n 0 ist, der Ligand L abwesend ist;
R1 oder R2 eine funktionelle Gruppe ist, die unabhängig aus der Gruppe ausgewählt wird, die aus Aminen, ungesättigten aromatischen carbocyclischen Gruppen, Carboxylaten, Estern, Ketonen, Halogenen und Nitrate besteht, wobei
ungesättigte aromatische carbocyclische Gruppen 6 bis 12 Kohlenstoffatome und einen einzelnen Ring oder mehrere kondensierte (anellierte) Ringe aufweisen, wobei mindestens ein Ring aromatisch ist und wobei die ungesättigte aromatische carbocyclische Gruppe unsubstituiert oder substituiert sein kann,
Carboxylat ein Anion bedeutet, das von einer Carbonsäure stammt, die ein Salz mit dem Palladium(II)-Kation bildet, Carboxylat stärker bevorzugt von einer niederen Carbonsäure, wie z. B. Essigsäure, stammt,
Ester einen Alkylester, bedeutet und
Keton ein Alkylketon, wie z. B. Acetylacetonat, bedeutet.

4. Verfahren nach Anspruch 3, wobei der Ligand L aus der Gruppe ausgewählt wird, die aus aromatischen und aliphatischen ein- und zweizähnigen Aminen (n = 1 oder 2) und ein- und zweizähnigen (n = 1 oder 2) Heteroarenen besteht, wobei
jede der aromatischen Amingruppen von einer Ammoniakgruppe stammt, in der ein, zwei oder drei Wasserstoffatome durch eine Arylgruppe substituiert sind,
jede der aliphatischen Amingruppen von einer Ammoniakgruppe stammt, in der ein, zwei oder drei Wasserstoffatome durch eine aliphatische Alkylgruppe substituiert sind, wobei
aliphatische Alkylgruppe einen Substituenten bedeutet, der eine verzweigte oder unverzweigte gesättigte Kohlenwasserstoffkette mit 1 bis 10 Kohlenstoffatomen, vorzugsweise 1 bis 4 Kohlenstoffatomen, aufweist, und
Arylgruppe einen ungesättigten aromatischen carbocyclischen Substituenten bedeutet, der 6 bis 12 Kohlenstoffatome aufweist und einen einzelnen Ring oder mehrere kondensierte Ringe aufweist, wobei mindestens ein Ring aromatisch ist, wobei die ungesättigte aromatische carbocyclische Gruppe unsubstituiert oder substituiert sein kann, oder
Arylgruppe eine Heteroarylgruppe bedeutet, die von einem monocyclischen, bicyclischen oder tricyclischen Heteroaren-Ringsystem abkommt, das einen, zwei oder drei aromatische Ringe enthält und mindestens ein Stickstoffatom in einem aromatischen Ring enthält, und die unsubstituiert oder substituiert sein kann, und
Heteroarengruppe ein monocyclisches, bicyclisches oder tricyclisches Heteroaren-Ringsystem bedeutet, das einen, zwei oder drei aromatische Ringe enthält und mindestens ein Stickstoffatom in einem aromatischen Ring enthält, und die unsubstituiert oder substituiert sein kann.

5. Verfahren nach einem von Anspruch 3 und 4, wobei der Palladiumkatalysator (I) aus der Gruppe ausgewählt wird, die aus Palladium(II)-acetat Pd(OAc)₂, Phenylpalladium(II)-acetat Pd(C₆H₅)(O₂CCH₃), Palladium(II)-acetylacetonat Pd(acac)₂ und Palladium(II)-nitrat Pd(NO₃)₂ besteht.

6. Verfahren nach Anspruch 1, wobei der Palladium(II)-Katalysator die allgemeine Formel (IA) aufweist,
LₙPd(II)R₂ (IA)
wobei die Definition von L die gleiche Bedeutung wie oben aufweist und R die gleiche Bedeutung wie R1 oder R2 oben aufweist und n 1 oder 2 bedeutet.

7. Verfahren nach einem von Anspruch 3 bis 6, wobei der Palladium(II)-Katalysator mit der Formel (I) aus der Gruppe ausgewählt wird, die besteht aus: Palladium(II)-Verbindung, immobilisiert auf Talcit, Palladium(II)-hydrotalcit-Komplexen, Palladium(II)-Verbindung-Pyridin-Komplexen und Palladium(II)-Verbindung-Triethylamin-(TEA)-Komplexen.

8. Verfahren nach Anspruch 7, wobei der Palladium(II)-Katalysator aus der Gruppe ausgewählt wird, die besteht aus: (py)₂Pd(OAC)₂, (py)₂Pd(acac)₂, (py)₂Pd(NO₃)₂, (Net₃)₂Pd(Oac)₂, (Net₃)₃Pd(Oac)₂, wobei py Pyridin bedeutet und et Ethyl bedeutet.

9. Verfahren nach einem der vorhergehenden Ansprüche, wobei der Schritt des Oxidierens von Betulin zu Betulonaldehyd oder Betulinaldehyd mittels Pd(II)-katalysierter Oxidation in Gegenwart von Disauerstoff in einem Lösemittel durchgeführt wird, das aus der Gruppe ausgewählt wird, die aus aromatischen Kohlenwasserstoffen, Ethern, halogenierten aliphatischen Kohlenwasserstoffen, H₂O oder Gemischen davon besteht.

10. Verfahren nach Anspruch 9, wobei das Lösemittel CH₂Cl₂, DMF oder THF, DMSO, H₂O, Perfluoralkan, z. B. Perfluordecalin, Toluol, Xylol oder Gemische davon ist.

11. Verfahren nach einem der vorhergehenden Ansprüche, wobei die Temperatur bei der Oxidation von Betulin 50 bis 120 °C, vorzugsweise etwa 80 °C, beträgt.

12. Verfahren nach einem der vorhergehenden Ansprüche, wobei der Palladiumkatalysator, nachdem sein Pd(II)-Anteil bei der Oxidation von Betulin zu Betulonaldehyd und/oder Betulinaldehyd zum Pd(0)-Anteil reduziert wurde, regeneriert wird.

13. Verfahren nach Anspruch 12, wobei der Palladiumkatalysator mittels N-Acetyl-L-cystein oder Mercaptophenylaminobut-2-enoatester regeneriert wird.

## Revendications

1. Procédé pour la préparation d'acide bétulonique ou d'acide bétulinique issu de la bétuline dans lequel le procédé comprend les étapes du processus suivantes :
(c1) l'oxydation de la bétuline en aldéhyde bétulonique en présence d'un catalyseur et d'un oxydant,
(c2) l'oxydation dudit aldéhyde bétulonique en acide bétulonique avec un système oxydant,
(c3) la réduction dudit acide bétulonique en acide bétulinique en utilisant un agent de réduction dans lequel
la réaction d'oxydation catalytique de la bétuline en aldéhyde bétulonique, à l'étape (c1), est réalisée simultanément, dans la même cuve de réaction, avec l'oxydation de l'aldéhyde bétulonique en acide bétulonique durant l'étape (c2)
ou
(d1) l'oxydation de la bétuline en aldéhyde bétulinique en présence d'un catalyseur et un oxydant,
(d2) l'oxydation dudit aldéhyde bétulinique en acide bétulinique avec un système oxydant, dans lequel
la réaction d'oxydation catalytique de la bétuline en aldéhyde bétulinique à l'étape (d1) est réalisée simultanément, dans la même cuve de réaction, avec l'oxydation de l'aldéhyde bétulinique en acide bétulinique durant l'étape (d2) et
- à l'étape (c1) ou (d1) l'oxydation catalytique de la bétuline est réalisée grâce à un catalyseur au Pd(II) en présence de dioxygène.

2. Procédé selon la revendication 1, dans lequel le dioxygène provient de l'oxygène atmosphérique.

3. Procédé selon la revendication 1 ou selon la revendication 2, dans lequel le catalyseur au Pd(II) est de la formule générale (I) 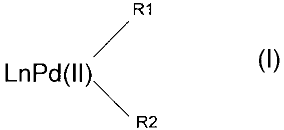 où
L est un ligand hétérocyclique ou aliphatique contenant au moins un ligand N d'atome d'azote,
n est un nombre entier quelconque allant de 0 à 2, dans le cas que n est égal à 0 le ligand L est absent ;
R1 ou R2 est un groupe fonctionnel choisi indépendamment parmi le groupe constitué d'amines, de groupes carbocycliques aromatiques insaturés, de carboxylates, d'esters, de cétones, d'halogènes et de nitrates, dans lequel
des groupes carbocycliques aromatiques insaturés ont de 6 à 12 atomes de carbone et un cycle unique ou divers cycles (fusionnés) condensés, dans lequel au moins un cycle est aromatique et où ledit groupe carbocyclique aromatique insaturé peut être non substitué ou substitué,
un carboxylate indique un anion issu d'un acide carboxylique qui forme un sel avec un cation de palladium(II), plus préférentiellement un carboxylate qui provient de l'acide carboxylique inférieur tel que l'acide acétique,
l'ester indique un ester d'alkyle, et
la cétone indique une cétone d'alkyle, telle que l'acétylacétonate.

4. Procédé selon la revendication 3, dans lequel le ligand L est choisi parmi le groupe constitué d'amines mono et bidentates aromatiques et aliphatiques (n=1 ou 2) et des hétéroarènes mono et bidentates (n=1 ou 2), où
chacun des groupes amines aromatiques provient d'un groupe ammoniac dans lequel un, deux ou trois atomes d'hydrogène(s) est/sont substitué(s) avec un groupe aryle,
chacun des groupes amines aliphatiques provient d'un groupe ammoniac dans lequel un, deux ou trois hydrogène(s) est/sont substitué(s) avec un groupe alkyle aliphatique, où
un groupe alkyle aliphatique indique un substituant ayant une chaîne d'hydrocarbures saturés non ramifiée ou ramifiée allant de 1 à 10 atomes de carbone, de préférence de 1 à 4 atomes de carbone et
un groupe aryle indique un substituant carbocyclique aromatique insaturé ayant de 6 à 12 atomes de carbone et ayant un cycle unique ou divers cycles condensés, dans lequel au moins un cycle est aromatique, où ledit groupe carbocyclique aromatique insaturé peut être non substitué ou substitué ou
un groupe aryle indique un groupe hétéroaryle qui est un dérivé d'un système de cycles hétéroarènes monocyclique, bicyclique, ou tricyclique contenant un, deux ou trois cycles aromatiques et contenant au moins un atome d'azote dans un cycle aromatique, et qui peut être non substitué ou substitué et
un groupe hétéroarène indique un système de cycles hétéroarènes monocyclique, bicyclique, ou tricyclique contenant un, deux ou trois cycles aromatiques et contenant au moins un atome d'azote dans un cycle aromatique, et qui peut être non substitué ou substitué.

5. Procédé selon l'une quelconque des revendications 3 ou 4, dans lequel le catalyseur au palladium (I) est choisi parmi le groupe constitué d'acétate de palladium (II) Pd(OAc)₂, acétate de phénylpalladium(II) Pd(C₆H₅)(O₂CCH₃), acétylacétonate de palladium(II) Pd(acac)₂ et nitrate de palladium(II) Pd(NO₃)₂.

6. Procédé selon la revendication 1, dans lequel le catalyseur au palladium (II) est de formule générale (IA).
LₙPd(II)R₂ (IA)
où la définition de L a le même sens que ci-dessus et R ont le même sens que R1 ou R2 ci-dessus et n indique 1 ou 2.

7. Procédé selon l'une quelconque des revendications 3 - 6, dans lequel le catalyseur au palladium(II) de la formule (I) est choisi parmi le groupe constitué de : composé de palladium(II) immobilisé sur talcite, complexes d'hydrotalcite de palladium (II), complexes de pyridine de composé de palladium(II) et complexes de triéthylamine (TEA) de composé de palladium (II).

8. Procédé selon la revendication 7, dans lequel le catalyseur au palladium (II) est choisi parmi le groupe constitué de : (py)₂Pd(OAC)₂, (py)₂Pd(acac)₂, (py)₂Pd(NO₃)₂, (Net₃)₂Pd(Oac)₂, (Net₃)₃Pd(Oac)₂, dans lequel py indique la pyridine et « et » indique l'éthyle.

9. Procédé selon l'une quelconque des revendications précédentes, dans lequel l'étape d'oxydation de la bétuline en aldéhyde bétulonique ou aldéhyde bétulinique avec l'oxydation de Pd(II) catalysé en présence de dioxygène est réalisée dans un solvant choisi parmi le groupe constitué d'hydrocarbures aromatiques, d'éthers, hydrocarbures aliphatiques halogénés, H₂O, ou des mélanges de ceux-ci.

10. Procédé selon la revendication 9, dans lequel le solvant est du CH₂Cl₂, du DMF ou du THF, du DMSO, H₂O, un perfluoroalcane par exemple du perfluorodécaline, du toluène, du xylène ou des mélanges de ceux-ci.

11. Procédé selon l'une quelconque des revendications précédentes, dans lequel la température pendant l'oxydation de la bétuline va de 50 - 120 °C, de préférence environ 80 °C.

12. Procédé selon l'une quelconque des revendications précédentes, dans lequel le catalyseur au palladium est régénéré après que sa fraction Pd(II) a été réduite en une fraction Pd(0) pendant l'oxydation de la bétuline en aldéhyde bétulonique et/ou en aldéhyde bétulinique.

13. Procédé selon la revendication 12, dans lequel le catalyseur au palladium est régénéré au moyen de N-acétyl-L-cystéine ou d'ester de mercaptophénylaminobut-2-énoate.
